# EUROPEAN PATENT SPECIFICATION

(11) **EP 3 222 591 B1**
(45) Date of publication and mention of the grant of the patent: **31.07.2019**
(21) Application number: 15820194.7
(22) Date of filing: 19.11.2015
(51) Int. Cl.: C02F 11/18, C02F 1/02, C12M 1/33

(54) **METHOD FOR THE CONTINUOUS THERMAL HYDROLYSIS OF ORGANIC MATTER, AND A SYSTEM SUITABLE FOR CARRYING OUT SAID METHOD**
METHODE FÜR DIE KONTINUIERLICHE THERMISCHE HYDROLYSE ORGANISCHER SUBSTANZ UND SYSTEM ZUR AUSFÜHRUNG DER METHODE
PROCÉDÉ POUR L'HYDROLYSE THERMIQUE EN CONTINU DE MATIÈRE ORGANIQUE ET INSTALLATION PERMETTANT LA MISE EN OEUVRE DE CE PROCÉDÉ

(30) Priority: 19.11.2014 ES 201431703
(43) Date of publication of application: 27.09.2017
(73) Proprietor: AQUATEC, PROYECTOS PARA EL SECTOR DEL AGUA, S.A.U., 28037 Madrid (ES)
(72) Inventor: ARAUZO PEREZ, Ivan, E-12004 CastellóN (ES); PERMUY VILA, Juan, E-12004 Castelló (ES)
(74) Representative: Sugrañes Patentes y Marcas
(86) International application number: PCT/ES2015/070825
(87) International publication number: WO 2016/079361

(56) References cited:
- WO-A1-96/09882
- WO-A1-2011/006854
- US-A1- 2004 192 980

## Description

### Technical field of the invention

The invention relates to a method for the continuous thermal hydrolysis of organic material, such as sludge having low solids content, as well as a plant suitable for implementing such method.

### Background of the invention

The method for the thermal hydrolysis of the sludge produced in wastewater treatment plants is a known process. The thermal hydrolysis process is based on subjecting the sludge to a pressure and temperature state for a given time period and causing a subsequent sudden decompression.

This hydrolyzed sludge presents a rupture of the cell membranes that is why it can be digested more easily by the bacteria during the subsequent digestion process, improving the performance thereof and making it possible to increase the biogas production.

The thermal hydrolysis process is an endothermic process, which requires an energy supply under conditions sufficient to reach both the temperature setpoint and the pressure setpoint that the reaction requires.

The pressure increase is carried out by pumping and has a known low consumption, however the heat energy supplied entails a high operating cost. That is why it is important to optimize the thermal hydrolysis processes at the level of design, in order to minimize the amount of primary energy needed. This improvement can be achieved with methods for recovering the energy dissipated during the sudden decompression and/or use of the energy dissipated with the hydrolyzed sludge.

An example of energy recovering is described in the patent document WO 2011/006854, disclosing a batch process wherein steam from a first pressure relieve tank is led to a preheating tank to heat the biomass afterwards lead to the reactors.

On the other hand, the thermal hydrolysis process carried out continuously favors the decrease of the plant size and consequently the investment cost thereof.

One aim of the present invention is a method by means of which investment costs as well as operative or working costs are minimized.

With regard to operative or working costs, actually there are known proposals essentially aimed to improve the use of energy in the hydrolysis method, which entails a reduction in operational cost.

This is the case for example, of the methods providing a sludge preheating stage and in which said sludge and the steam employed to reach the conditions in the reactor are at the same temperature, which means that a liquid (sludge) and a saturated gas (steam) coexist in both, the preheater and the reactor.

The patent document US 2004/0192980 describes a continuous process wherein steam and gases are diverted back to a feed storage tank to assist in heating the influent feed.

On one hand, one of the main disadvantages of this type of processes is that a small decrease in the pressure causes a vaporization of one portion of the liquid phase. For example, in the transfer pumps used with this sludge there are points where the pressure momentarily decreases causing a vaporization and a subsequent collapse as soon as the pressure increases again due to the own pump process. This phenomenon, known as cavitation, decreases the reliability of the pieces of equipment of the plant.

This entails a series of technical problems, which make the implementation of such methods unfeasible at an industrial level, since the pieces of equipment are not prepared for operating at the points and under the working conditions the process demands or would require.

On the other hand, during the sludge preheating stage, the pressure at which this preheating is carried out has to be lower than the pressure of the subsequent sudden decompression, which is carried out in a chamber or flash tank. This fact makes it necessary after the preheating stage to pump the sludge, which as it has been mentioned earlier, is in a saturation state and generates cavitation problems in the equipment.

Another relevant prior art is WO 00/73221.

Therefore, one additional aim of the present invention is a method that can overcome the drawbacks of the prior art.

### Description of the invention

The method for the continuous thermal hydrolysis of organic material according to the invention is defined in the appended claim 1. This method comprises at least the stages of:
a) pressurizing starting organic material at a pressure setpoint P1;
b) inline mixing pressurized organic material with steam in a liquid-steam mixing valve to obtain a mixture at a controlled temperature setpoint T1, selected below the steam saturation temperature of the mixture to ensure that the resulting mixture is in a liquid phase;
c) continuously introducing the mixture obtained, which is in a liquid phase, into a hydrolysis reactor;
d) continuously extracting hydrolyzed mixture from the hydrolysis reactor; and
e) suddenly depressurizing said hydrolyzed mixture separating a liquid fraction from a steam fraction, which is recovered;
wherein the steam employed to produce the mixture in stage b) is obtained by mixing new steam with steam recovered in operation e) which is recompressed. The working pressure in the reactor is kept above the steam saturation pressure at the temperature at which the process is carried out, ensuring that the mixture remains in a liquid phase in the reactor.

The elimination of one preheating stage and the fact of maintaining the mixture in a liquid phase makes it possible to overcome the drawbacks of known methods. Herein, liquid phase means that the mixture will not contain steam although it may contain some volatile compounds in smaller amounts, the nature of which will depend on the organic material to be treated.

Advantageously, the method of the invention is a reliable method, given that the state of the current technology makes the pieces of equipment needed for the implementation thereof, work within their normal operating intervals.

Thus, in a preferred variant of the invention the starting organic material is at room temperature.

Unlike known methods, the energy recovery is carried out employing steam recompression methods.

In a variant of the method, the recovered steam is recompressed employing steam recompressing methods, either mechanical, thermal or a combination of both.

According to the invention, the steam employed for producing the mixture in stage b) is obtained by mixing new steam with steam recovered from stage e) which is recompressed.

The invention provides for the recovered steam, to be recompressed in a first step, by a steam compressor, which rises the pressure thereof between 4 and 6 bar; and subsequently in a second step, the mechanically recompressed steam is thermally compressed by mixing it with new steam, the differential pressure provided in this second step being between 1 and 1.5 bar.

According to the invention, the inline mixture of pressurized organic material and steam is carried out in a liquid-steam mixing valve.

Preferably, the temperature setpoint T1, which is comprised between 130 and 190 °C, is between 5 and 10°C lower than the steam saturation temperature of the resulting mixture for pressure P1, which is comprised between 3 and 16 bar.

The stay time of the mixture in the hydrolysis reactor is preferably between 5 and 60 min.

In a variant, stage e) of depressurization is carried out by means of a regulating valve causing a differential pressure ranging between 4 and 8 bar.

In a variant, the liquid fraction obtained in stage e) of depressurization is at a pressure sufficient to pump by pressure difference said liquid fraction to digestion tanks.

According to another aspect of the invention, a plant suitable for implementing the claimed method is disclosed.

This plant, especially suitable for sludges having low solids content, comprises
- means for feeding the starting organic material, which comprise a duct and a supply pump that can rise the circulating organic material to an absolute pressure setpoint P1 between 0.3 and 1.6 Mpa (3 and 16 bar);
- a mixing device comprising a liquid-steam mixing valve, connected to the feeding means and a steam source, suitable for mixing the pressurized organic material with the steam and obtaining a mixture in liquid state at a controlled temperature setpoint T1, between 130° and 190 °C, below the steam saturation temperature of the obtained mixture;
- a hydrolysis reactor with at least an inlet and an outlet, the inlet being connected at the outlet of the mixing device and the reactor being prepared to maintain the mixture under absolute pressure conditions ranging between 3 and 16 bar and at a temperature ranging between 130 and 180 °C during the stay thereof inside the reactor, wherein the selected pressure conditions are above the saturation pressure at the temperature at which the process is carried out to ensure that the pressurized organic material with the steam remains in a liquid phase in the reactor (9);
- a regulating valve, connected at the outlet of the hydrolysis reactor, capable of promoting the decompression of the hydrolyzed mixture coming out from the reactor;
- an expansion chamber, connected to the regulating valve, where a liquid fraction is separated from a steam fraction, which is recovered;
- at least one recompression device of the steam recovered in the expansion chamber; and
- at least one second mixing device for adding new steam to the recovered and recompressed steam, the result being supplied by the steam source that is connected to that effect to the mixing device.

### Brief description of the drawings

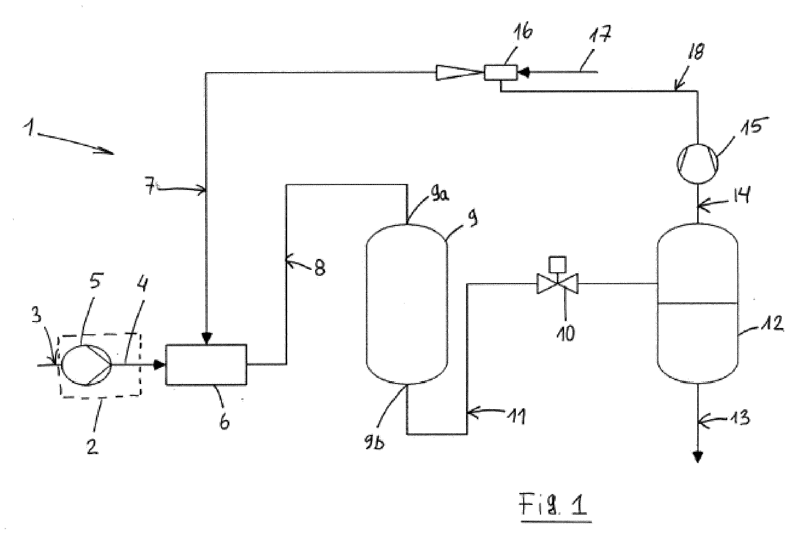

Fig. 1, is a schema of a plant according to the invention.

### Detailed description of one embodiment

The plant 1 of Fig. 1 is an example of a plant for the continuous thermal hydrolysis of organic material, especially suitable for sludge having low solids content.

With reference to the schema of Fig. 1, the stages of the method and the pieces of equipment included in the exemplary plant for the implementation thereof are indicated below.

The organic material to be hydrolyzed, in the example a starting sludge 3 at room temperature, is fed by means of feeding means 2, which in the example comprise a duct 4 and a supply pump 5 capable of rising the circulating sludge to an absolute pressure setpoint P1 between 3 and 16 bar.

The plant 1 comprises downstream the supply pump 5 a mixing device 6, connected on one side to the feeding means 2 and on the other side to a steam source 7 at a pressure Pv higher or equal to the pressure setpoint P1. The mixing device 6, which is a liquid-steam mixing valve, mixes the pressurized organic material with steam, thus obtaining a mixture 8, which contains sludge in a heated liquid state.

A characteristic of the method is that said heating is carried out by inline mixing, and controlling that the temperature of the obtained mixture 8 is a controlled temperature setpoint T1 lower than the steam saturation temperature of the mixture, so as to obtain a mixture 8 without steam, essentially in a liquid state. In the example, the temperature setpoint T1 may be between 5 and 10 °C lower than the saturation temperature for pressure P1, said temperature setpoint T1 being comprised between 130° and 190 °C.

The mixture 8, in liquid phase, is introduced in a hydrolysis reactor 9 through an inlet 9a, provided for such purposes on the upper portion thereof, and is extracted through an outlet 9b, provided in the lower portion of the reactor. In the example, the balance of the material being introduced in and extracted from the reactor 9 is zero.

The working pressure in the reactor 9 is kept above the saturation pressure at the temperature at which the process is carried out, such that the sludge and steam mixture 8 remains in a liquid phase.

The dimensions of the reactor 9 are selected such that the time of stay of the mixture 8 is the appropriate one, preferably being comprised between 5 and 60 min.

Additionally, the reactor 9 is internally provided with a stirring mechanism to prevent eventual problems during the startup and shutdown phases of the plant 1.

A regulating valve 10, connected at the outlet 9b of the hydrolysis reactor 9, brings about the sudden decompression of the hydrolyzed mixture 11 extracted from the reactor 9.

In this stage of sudden decompression, the breaking of the cell walls of the sludge contained in the hydrolyzed mixture 11 takes place. The decompression is carried out using a regulating valve that causes a pressure difference ranging between 4 and 8 bar relative to the pressure setpoint P1, and is confined in an expansion chamber 12, connected to the regulating valve 10, wherein a liquid fraction 13 is separated from a steam fraction 14, which is recovered.

In the example, this recovered steam 14 is subjected in a first step to a mechanical recompression by means of a recompression device 15, in the form of a compressor, the differential pressure provided in this step being between 4 and 6 bar. Then, in a second step the mechanically recompressed steam 18 is thermally compressed by mixing it, in a mixing device 16 based on the Venturi effect, with new steam 17, the differential pressure provided in this second step being between 1 and 1.5 bar, thus obtaining the steam source 7 that feeds the mixing device 6.

## Claims

1. A method for the continuous thermal hydrolysis of organic material, which comprises at least the stages of:
a) pressurizing starting organic material (3) at a pressure setpoint P1;
b) inline mixing pressurized organic material with steam (7) in a liquid-steam mixing valve to obtain a mixture (8) at a controlled temperature setpoint T1, selected below the steam saturation temperature of the mixture to ensure that the mixture is in a liquid phase;
c) continuously introducing the mixture (8) obtained, which is in a liquid phase, in a hydrolysis reactor (9);
d) continuously extracting hydrolyzed mixture (11) from the hydrolysis reactor (9); and
e) suddenly depressurizing said hydrolyzed mixture separating a liquid fraction (13) from a steam fraction, which is recovered (14);
wherein the steam (7) employed to produce the mixture in stage b) is obtained by mixing new steam (17) with steam recovered (14) in operation e) which is recompressed, and wherein the working pressure in the reactor (9) is kept above the steam saturation pressure at the temperature at which the process is carried out, ensuring that the mixture (8) remains in a liquid phase in the reactor (9).

2. The method according to claim 1, **characterized in that** the starting organic material (3) is at room temperature.

3. The method according to any one of the previous claims, **characterized in that** the recovered steam (14) is recompressed employing steam recompressing methods either mechanical, thermal or a combination of both.

4. The method according to claim 3, **characterized in that** the recovered steam (14) is recompressed in a first step, by a steam compressor, which rises the pressure thereof between 4 and 6 bar; and subsequently in a second step the mechanically recompressed steam (18) is thermally compressed by mixing it with new steam (17), the differential pressure provided in this second step being between 1 and 1.5 bar.

5. The method according to any one of the previous claims, **characterized in that** the temperature setpoint T1 is between 5 and 10 °C lower than the steam saturation temperature of the mixture for pressure P1.

6. The method according to any one of the previous claims, **characterized in that** the stay time of the mixture in the hydrolysis reactor (9) is between 5 and 60 min.

7. The method according to any one of the previous claims, **characterized in that** stage e) of depressurization is carried out by means of a regulating valve causing a differential pressure ranging between 4 and 8 bar.

8. The method according to any one of the previous claims, **characterized in that** the liquid fraction (13) obtained in stage e) of depressurization is at a pressure sufficient to pump by pressure difference said liquid fraction (13) to digestion tanks.

9. The method according to any one of the previous claims, **characterized in that** the pressure setpoint P1 is between 3 and 16 bar.

10. The method according to any one of the previous claims, **characterized in that** the temperature setpoint T1 is between 130 and 190 °C.

11. A plant (1) for the continuous thermal hydrolysis of organic material, especially suitable for sludge having low solids content, comprising
- means (2) for feeding the starting organic material (3), which comprise a duct (4) and a supply pump (5) capable of rising the circulating organic material to an absolute pressure setpoint P1 between 3 and 16 bar;
- a mixing device (6) comprising a liquid-steam mixing valve, connected to the feeding means (2) and a steam source (7), suitable for mixing the pressurized organic material with the steam and obtaining a mixture (8) in a liquid state at a controlled temperature setpoint T1, between 130° and 190 °C, below the steam saturation temperature of the mixture;
- a hydrolysis reactor (9) with at least an inlet (9a) and an outlet (9b), the inlet (9a) being connected at the outlet of the mixing device (6) and the reactor being prepared to maintain the mixture (18) under absolute pressure conditions ranging between 3 and 16 bar and at a temperature ranging between 130 and 180 °C during the stay thereof inside the reactor, wherein the selected pressure conditions are above the steam saturation pressure at the temperature at which the process is carried out to ensure that the pressurized organic material with the steam remains in a liquid phase in the reactor (9);
- a regulating valve (10), connected at the outlet (9b) of the hydrolysis reactor (9), capable of promoting the decompression of the hydrolyzed mixture (11) coming out from the reactor;
- an expansion chamber (12), connected to the regulating valve (10), where a liquid fraction (13) is separated from a steam fraction (14), which is recovered;
- at least one recompression device (15) of the steam (14) recovered in the expansion chamber (12); and
- at least one second mixing device (16) for adding new steam (17) to the recovered and recompressed steam (18) the result being supplied by the steam source (7) connected to that effect to the mixing device (6).

## Patentansprüche

1. Methode für die kontinuierliche thermische Hydrolyse organischer Substanz, welche mindestens die folgenden Schritte umfasst:
a) das Beaufschlagen mit Druck der anfänglichen organischen Substanz (3) bei einem Drucksollwert P1;
b) das Inline-Mischen von mit Druck beaufschlagter organischer Substanz mit Dampf (7) in einem Flüssigkeit-Dampf-Mischventil zum Erhalten einer Mischung (8) bei einem gesteuerten Temperatursollwert T1, ausgewählt unter der Dampfsättigungstemperatur der Mischung, um zu gewährleisten, dass die Mischung in einer Flüssigphase ist;
c) das kontinuierliche Einführen der erhaltenen Mischung (8), welche in einer Flüssigphase ist, in einen Hydrolysereaktor (9);
d) das kontinuierliche Entnehmen der hydrolysierten Mischung (11) aus dem Hydrolysereaktor (9); und
e) das plötzliche Druckentlasten der genannten hydrolysierten Mischung unter Trennung einer Flüssigkeitsfraktion (13) von einer Dampffraktion, welche zurückgewonnen wird (14);
wobei der Dampf (7), welcher dazu verwendet wird, die Mischung in Schritt b) herzustellen, derart erhalten wird, dass neuer Dampf (17) mit im Vorgang e) zurückgewonnenem Dampf (14), welcher rekomprimiert ist, gemischt wird, und wobei der Arbeitsdruck im Reaktor (9) über dem Dampfsättigungsdruck gehalten wird, bei der Temperatur, bei der das Verfahren ausgeführt wird, so dass gewährleistet wird, dass die Mischung (8) im Reaktor (9) in einer Flüssigphase bleibt.

2. Methode nach Anspruch 1, **dadurch gekennzeichnet, dass** die anfängliche organische Substanz (3) bei Raumtemperatur ist.

3. Methode nach einem der vorhergehenden Ansprüche, **dadurch gekennzeichnet, dass** der zurückgewonnene Dampf (14) rekomprimiert wird, unter Verwendung von Dampfrekomprimierungsmethoden, entweder mechanisch, thermisch oder eine Kombination derselben.

4. Methode nach Anspruch 3, **dadurch gekennzeichnet, dass** der zurückgewonnene Dampf (14) in einem ersten Schritt rekomprimiert wird, mittels eines Dampfkompressors, welcher den Druck desselben zwischen 4 und 6 bar erhöht; und anschließend in einem zweiten Schritt der mechanisch rekomprimierte Dampf (18) thermisch komprimiert wird, indem er mit neuem Dampf (17) gemischt wird, wobei der in diesem zweiten Schritt bereitgestellte Differenzialdruck zwischen 1 und 1,5 bar liegt.

5. Methode nach einem der vorhergehenden Ansprüche, **dadurch gekennzeichnet, dass** der Temperatursollwert T1 zwischen 5 und 10°C unter der Dampfsättigungstemperatur der Mischung für den Druck P1 liegt.

6. Methode nach einem der vorhergehenden Ansprüche, **dadurch gekennzeichnet, dass** die Verweilzeit der Mischung im Hydrolysereaktor (9) zwischen 5 und 60 min liegt.

7. Methode nach einem der vorhergehenden Ansprüche, **dadurch gekennzeichnet, dass** Schritt e) der Druckentlastung mittels eines Regelventils ausgeführt wird, so dass ein Differenzialdruck hervorgerufen wird, welcher zwischen 4 und 8 bar liegt.

8. Methode nach einem der vorhergehenden Ansprüche, **dadurch gekennzeichnet, dass** die in Schritt e) der Druckentlastung erhaltenen Flüssigkeitsfraktion (13) bei einem Druck ist, welcher ausreichend ist, um die genannte Flüssigkeitsfraktion (13) mittels Druckunterschied zu Faulbehältern zu pumpen.

9. Methode nach einem der vorhergehenden Ansprüche, **dadurch gekennzeichnet, dass** der Drucksollwert P1 zwischen 3 und 16 bar liegt.

10. Methode nach einem der vorhergehenden Ansprüche, **dadurch gekennzeichnet, dass** der Temperatursollwert T1 zwischen 130 und 190°C liegt.

11. Anlage (1) für die kontinuierliche thermische Hydrolyse organischer Substanz, besonders für Schlamm geeignet, welcher einen niedrigen Feststoffgehalt aufweist, welche Folgendes umfasst
- Mittel (2) zum Einspeisen anfänglicher organischer Substanz (3), welche eine Leitung (4) und eine Zuführungspumpe (5) umfassen, welche in der Lage ist, die umlaufende organische Substanz bis zu einem absoluten Drucksollwert P1 zwischen 3 und 16 bar zu erhöhen;
- eine Mischvorrichtung (6) umfassend ein Flüssigkeit-Dampf-Mischventil, welche mit den Einspeisemitteln (2) und einer Dampfquelle (7) verbunden ist, welche dazu geeignet ist, die mit Druck beaufschlagter organischer Substanz mit dem Dampf zu mischen und eine Mischung (8) in einem flüssigen Zustand bei einem gesteuerten Temperatursollwert T1, zwischen 130° und 190°C, unter der Dampfsättigungstemperatur der Mischung zu erhalten;
- einen Hydrolysereaktor (9) mit mindestens einem Eingang (9a) und einem Ausgang (9b), wobei der Eingang (9a) am Ausgang der Mischvorrichtung (6) verbunden ist und wobei der Reaktor dazu vorbereitet ist, die Mischung (18) unter absoluter Druckbedingungen, welche zwischen 3 und 16 bar liegen, und bei einer Temperatur, welche zwischen 130 und 180°C liegt, während der Verweilzeit derselben innerhalb des Reaktors zu halten, wobei die ausgewählten Druckbedingungen über dem Dampfsättigungsdruck bei der Temperatur, bei welcher das Verfahren ausgeführt wird, sind, um zu gewährleisten, dass die mit Druck beaufschlagter organischer Substanz mit dem Dampf in einer Flüssigphase im Reaktor (9) bleibt;
- ein Regelventil (10), am Ausgang (9b) des Hydrolysereaktors (9) verbunden, welches in der Lage ist, die Dekompression der hydrolysierten Mischung (11), welche aus dem Reaktor herauskommt, fördert;
- eine Expansionskammer (12), mit dem Regelventil (10) verbunden, in welcher eine Flüssigkeitsfraktion (13) von einer Dampffraktion (14) getrennt wird, welche zurückgewonnen wurde;
- mindestens eine Rekompressionsvorrichtung (15) des in der Expansionskammer (12) zurückgewonnenen Dampfs (14); und
- mindestens eine Mischvorrichtung (16) zum Hinzufügen von neuem Dampf (17) zum zurückgewonnenen und rekomprimierten Dampf (18), wobei das Ergebnis von der Dampfquelle (7), welche zu diesem Zweck mit der Mischvorrichtung (6) verbunden ist, zugeführt wird.

## Revendications

1. Procédé d'hydrolyse thermique en continu de matière organique, qui comprend au moins deux étapes de :
a) pressurisation de la matière organique de départ (3) à une pression de consigne P1 ;
b) mélange en ligne de la matière organique pressurisée avec de la vapeur (7) dans une vanne de mélange liquide-vapeur pour obtenir un mélange (8) à une température contrôlée de consigne T1, choisi en dessous de la température de saturation en vapeur du mélange pour assurer que le mélange est en phase liquide;
c) introduction en continue du mélange (8) obtenu, qui est en phase liquide, dans un réacteur d'hydrolyse (9);
d) extraction en continue du mélange hydrolysé (11) du réacteur d'hydrolyse (9); et
e) dépressurisation brusque dudit mélange hydrolysé séparant une fraction liquide (13) d'une fraction vapeur, qui est récupérée (14);
dans lequel la vapeur (7) employée pour produire le mélange dans l'étape b) est obtenue en mélangeant une nouvelle vapeur (17) avec la vapeur récupérée (14) de l'étape e) qui est récompressée, et dans lequel la pression de travail dans le réacteur (9) est maintenue au-dessus de la pression de saturation de la vapeur à la température à laquelle le procédé est mis en oeuvre, en assurant que le mélange (8) demeure en phase liquide dans le réacteur (9).

2. Procédé selon la revendication 1, **caractérisé en ce que** la matière organique de départ (3) est à la température ambiante.

3. Procédé selon l'une quelconque des revendications précédentes, **caractérisé en ce que** la vapeur récupérée (14) est recompressée en employant des procédés de recompression de vapeur soit mécanique, thermique ou bien une combinaison des deux.

4. Procédé selon la revendication 3, **caractérisé en ce que** la vapeur récupérée (14) est recompressée dans une première étape, par un compresseur de vapeur, qui élève sa pression entre 4 et 6 bar; et ensuite dans une seconde étape la vapeur mécaniquement recompressée (18) est compressée de manière thermique en la mélangeant avec une nouvelle vapeur (17), la pression différentielle prévue dans cette seconde étape étant entre 1 et 1,5 bar.

5. Procédé selon l'une quelconque des revendications précédentes, **caractérisé en ce que** la température de consigne T1 est entre 5 et 10°C inférieure à la température de saturation de vapeur du mélange pour la pression P1.

6. Procédé selon l'une quelconque des revendications précédentes, **caractérisé en ce que** le temps de séjour du mélange dans le réacteur d'hydrolyse (9) est entre 5 et 60 minutes.

7. Procédé selon l'une quelconque des revendications précédentes, **caractérisé en ce que** l'étape e) de dépressurisation est mise en oeuvre par le biais d'une vanne de régulation provoquant une pression différentielle allant de 4 à 8 bar.

8. Procédé selon l'une quelconque des revendications précédentes, **caractérisé en ce que** la fraction liquide (13) obtenue dans l'étape e) de dépressurisation est à une pression suffisante de pompage par différence de pression de ladite fraction liquide (13) dans des cuves de digestion.

9. Procédé selon l'une quelconque des revendications précédentes, **caractérisé en ce que** la pression de consigne P1 est entre 3 et 16 bar.

10. Procédé selon l'une quelconque des revendications précédentes, **caractérisé en ce que** la température de consigne T1 est entre 130 et 190°C.

11. Installation (1) pour l'hydrolyse thermique en continu de matière organique, spécialement appropriée pour des boues ayant une faible teneur en solides, comprenant
- des moyens (2) d'alimentation de la matière organique de départ (3), qui comprend un conduit (4) et une pompe d'alimentation (5) apte à élever la matière organique en circulation à une pression absolue de consigne P1 entre 3 et 16 bar;
- un dispositif de mélange (6) comprenant une vanne de mélange liquide-vapeur, connectée aux moyens d'alimentation (2) et une source de vapeur (7), appropriée pour mélanger la matière organique pressurisée avec la vapeur, et obtenir un mélange (8) en état liquide à une température contrôlée de consigne T1, entre 130° et 190°C, sous la température de saturation de vapeur du mélange;
- un réacteur d'hydrolyse (9) avec au moins une entrée (9a) et une sortie (9b), l'entrée (9a) étant connectée à la sortie du dispositif de mélange (6) et le réacteur étant préparé pour maintenir le mélange (18) sous des conditions de pression absolue allant de 3 à 16 bar et à une température allant de 130 à 180°C pendant son séjour au sein du réacteur, dans lequel les conditions de pression choisies sont au-dessus de la pression de saturation de vapeur à la température à laquelle le procédé est mis en oeuvre pour assurer que la matière organique pressurisée avec la vapeur demeure en phase liquide dans le réacteur (9);
- une vanne de régulation (10), connectée à la sortie (9b) du réacteur d'hydrolyse (9), apte à faciliter la décompression du mélange hydrolysé (11) sortant du réacteur;
- une chambre d'expansion (12), connectée à la vanne de régulation (10), où une fraction liquide (13) est séparée d'une fraction vapeur (14), qui est récupérée;
- au moins un dispositif de recompression (15) de la vapeur (14) récupérée dans la chambre d'expansion (12); et
- au moins un second dispositif de mélange (16) pour ajouter la nouvelle vapeur (17) à la vapeur récupérée et recompressée (18), le résultat étant alimenté par la source de vapeur (7) connectée à cet effet au dispositif de mélange (6).
